# EUROPEAN PATENT SPECIFICATION

(11) **EP 1 100 560 B1**
(45) Date of publication and mention of the grant of the patent: **16.01.2008**
(21) Application number: 99935882.3
(22) Date of filing: 23.07.1999
(51) Int. Cl.: A61M 1/32, A61M 25/00, B01F 3/04

(54) **MEDICAL APPARATUS FOR THE PREPARATION AND DELIVERY OF GAS-ENRICHED FLUIDS**
MEDIZINISCHE VORRICHTUNG FÜR DIE HERSTELLUNG UND ABGABE EINER MIT GAS ANGEREICHERTEN FLÜSSIGKEIT
APPAREIL MEDICAL POUR PREPARER ET APPORTER DES FLUIDES ENRICHIS EN GAZ

(30) Priority: 24.07.1998 US 122438; 24.07.1998 US 122143
(43) Date of publication of application: 23.05.2001
(73) Proprietor: TherOx, Inc., Irvine, CA 92614-5846 (US); WAYNE STATE UNIVERSITY, Detroit, MI 48202 (US)
(72) Inventor: DIVINO, Vincent, Jr., Mission Viejo, CA 92692 (US); FOERSTER, Seth, A., San Clemente, CA 92672 (US); GESSERT, James, S., Colorado Springs, CO 80906 (US); MEST, Robert, A., Long Beach, CA 90808 (US); ZALESKY, Paul, J., Huntington Beach, CA 92646 (US); SPEARS, James, R., Bloomfield Hills, MI 48304 (US)
(74) Representative: Müller-Boré & Partner Patentanwälte
(86) International application number: PCT/US1999/016743
(87) International publication number: WO 2000/004943

(56) References cited:
- WO-A-95/13843
- WO-A-96/17565
- WO-A-99/08733
- US-A- 4 664 680
- US-A- 4 802 650
- US-A- 5 211 627

## Description

### FIELD OF THE INVENTION

The present invention relates generally to an apparatus for the preparation and delivery of gas-enriched fluids to gas-depleted locations, and more particularly, to a system for the preparation and delivery of physiologic solutions for treating conditions such as tissue ischemia and post-ischemic tissues, including, *inter alia,* a catheter for delivering oxygen-enriched blood to specific locations within a patient's body.

### BACKGROUND OF THE INVENTION

Oxygen is a crucial nutrient for human cells. Cell damage may result from oxygen deprivation for even brief periods of time, which may lead to organ dysfunction or failure. For example, heart attack and stroke victims experience blood flow obstructions or diversions that prevent oxygen from being delivered to the cells of vital tissues. Without oxygen, the heart and brain progressively deteriorate. In severe cases death results from complete organ failure. Less severe cases typically involve costly hospitalization, specialized treatments and lengthy rehabilitation.

Blood oxygen levels may be described in terms of the partial pressure of the oxygen dissolved in the blood (pO₂). Typically, for arterial blood, normal blood oxygen levels (i.e., normoxia or normoxemia) range from 90-110 mm Hg (12-15 kPA). Hypoxemic blood (i.e., hypoxemia) is arterial blood with a pO₂ less than 90 mm Hg (12kPA). Hyperoxic blood (i.e., hyperoxemia or hyperoxia) is arterial blood with a pO₂ greater than 400 mm Hg (53kPA) (see Cason et. al (1992), Effects of High Arterial Oxygen Tension on Function, Blood Flow Distribution, and Metabolism in Ischemic Myocardium, Circulation, Vol. 85, No. 2, pp. 828-838), but less than 760 mm Hg (see Shandling et al. (1997), Hyperbaric Oxygen and Thrombolysis in Myocardial Infarction: The "HOT MI" Pilot Study, American Heart Journal Vol. 134, No. 3, pp. 544-550). Hyperbaric blood is arterial blood with a pO₂ greater than 760 mm Hg (101kPA). Venous blood typically has a pO₂ level less than 90 mm Hg (12kPA). In the average adult, for example, normal venous blood oxygen levels range generally from 40 mm Hg to 70 mm Hg (5 to 9 kPA).

Blood oxygen levels also might be described in terms of hemoglobin saturation levels. For normal arterial blood, hemoglobin saturation is about 97% and varies only slightly as pO₂ levels increase. For normal venous blood, hemoglobin saturation is about 75%.

In patients who suffer from acute myocardial infarction, if the myocardium is deprived of adequate levels of oxygenated blood for a prolonged period of time, irreversible damage to the heart can result. Where the infarction is manifested in a heart attack, the coronary arteries fail to provide adequate blood flow to the heart muscle.

Treatment of acute myocardial infarction or myocardial ischemia often comprises performing angioplasty or stenting of the vessels to compress, ablate or otherwise treat the occlusion(s) within the vessel walls. For example, a successful angioplasty increases the size of the vessel opening to allow increased blood flow.

Even with the successful treatment of occluded vessels, a risk of tissue injury may still exist. During percutaneous transluminal coronary angioplasty (PTCA), the balloon inflation time is limited by the patient's tolerance to ischemia caused by the temporary blockage of blood flow through a vessel during balloon inflation. Reperfusion injury also may result, for example, due to slow coronary reflow or no reflow following angioplasty.

For some patients angioplasty procedures are not an attractive option for the treatment of vessel blockages. Such patients typically are at increased risk of ischemia for reasons such as, poor left ventricular function, lesion type and location, or the amount of the myocardium at risk. The treatment options for such patients thus include more invasive procedures such as coronary bypass surgery.

To reduce the risk of tissue injury typically associated with treatments of acute myocardial infarction and myocardial ischemia, it is usually desirable to deliver oxygenated blood or oxygen-enriched fluids to at-risk tissues. Tissue injury is minimized or prevented by the diffusion of the dissolved oxygen from the blood or fluids to the tissue and/or blood perfusion that removes metabolites and that provides other chemical nutrients.

In some cases, the desired treatment of acute myocardial infarction and myocardial ischemia includes perfusion of oxygenated blood or oxygen-enriched fluids. During PTCA, for example, tolerated balloon inflation time may be increased by the concurrent introduction of oxygenated blood into the patient's coronary artery. Increased blood oxygen levels also may cause the normally perfused left ventricular cardiac tissue into hypercontractility to further increase blood flow through the treated coronary vessels.

The infusion of oxygenated blood or oxygen-enriched fluids also may be continued following the completion of PTCA treatment or other procedures (e.g. surgery) wherein cardiac tissue "stunning" with associated function compromise has occurred. In some cases continued infusion may accelerate the reversal of ischemia and facilitate recovery of myocardial function.

Conventional methods for the delivery of oxygenated blood or oxygen-enriched fluids to at-risk tissues involve the use of blood oxygenators. Such procedures generally involve withdrawing blood from a patient, circulating it through an oxygenator to increase blood oxygen concentration, and then delivering the blood back to the patient. One example of a commercially available blood oxygenator is the Maxima blood oxygenator manufactured by Medtronic, Inc., Minneapolis, Minnesota.

There are drawbacks, however, to the use of a conventional oxygenator in an extracorporeal circuit for oxygenating blood. Such systems typically are costly, complex and difficult to operate. Often a qualified perfusionist is required to prepare and monitor the system.

Conventional oxygenator systems also typically have a large priming volume, i.e., the total volume of blood contained within the oxygenator, tubing and other system components, and associated devices. It is not uncommon in a typical adult patient case for the oxygenation system to hold more than one to two liters of blood. Such large priming volumes are undesirable for many reasons. For example, in some cases a blood transfusion may be necessary to compensate for the blood temporarily lost to the oxygenation system because of its large priming volume. Heaters often must be used to maintain the temperature of the blood at an acceptable level as it travels through the extracorporeal circuit. Further, conventional oxygenator systems are relatively difficult to turn on and off. For instance, if the oxygenator is turned off, large stagnant pools of blood in the oxygenator might coagulate.

In addition, with extracorporeal circuits including conventional blood oxygenators there is a relatively high risk of inflammatory cell reaction and blood coagulation due to the relatively slow blood flow rates and the large blood contact surface area. A blood contact surface area of about 1-2 m² and velocity flows of about 3 cm/s are not uncommon with conventional oxygenator systems. Thus, relatively aggressive anti-coagulation therapy, such as heparinization, is usually required as an adjunct to using the oxygenator.

Perhaps one of the greatest disadvantages to using conventional blood oxygenation systems is that the maximum partial pressure of oxygen (pO₂) that can be imparted to blood with commercially available oxygenators is about 500 mm Hg. Thus, blood pO₂ levels near or above 760 mm Hg cannot be achieved with conventional oxygenators.

Some experimental studies to treat myocardial infarction have involved the use of hyperbaric oxygen therapy. See, e.g., Shandling et al. (1997), Hyperbaric Oxygen and Thrombolysis in Myocardial Infarction: The "HOT MI" Pilot Study, American Heart Journal. Vol. 134, No. 3, pp. 544-550. These studies generally have involved placing patients in chambers of pure oxygen pressurized at up to 2 atmospheres, resulting in systemic oxygenation of patient blood up to a pO₂ level of about 1200 mm. Hg. However, use of hyperbaric oxygen therapy following restoration of coronary artery patency in the setting of an acute myocardial infarction is not practical. Monitoring critically ill patients in a hyperbaric oxygen chamber is difficult. Many patients become claustrophobic. Ear damage may occur. Further, treatment times longer than 90 minutes cannot be provided without concern for pulmonary oxygen toxicity.

For these reasons, the treatment of regional organ ischemia generally has not been developed clinically. Thus, there remains a need for a simple and convenient system for delivering oxygen-enriched blood and other fluids to patients for the localized prevention of ischemia and the treatment of post-ischemic tissue and organs.

US 3 512 517 discloses an apparatus for monitoring blood glucose concentration comprising a catheter and pump to withdraw sampled blood from a patient's vein.

US 5 211 627 discloses a catheter for infusion of aerated liquid comprising an air lumen and a liquid lumen connected by means of an orifice. Accordingly, air delivered to the air lumen can be mixed with the liquid delivered to the liquid lumen within the lumen.

WO 99/08733 discloses an apparatus for increasing gas concentration in blood comprising a blood pump for withdrawing blood, means for the delivery of oxygen-supersaturated fluid and a chamber as a combining means for combining blood with the oxygen-supersaturated fluid.

US 5 394 732 discloses an apparatus for ultrasonic detection of air bubbles having a transmitter for transmitting a signal through the fluid and a receiver for receiving the transmitted signal. The presence of bubbles is determined on the basis of a comparison between an expected and received attenuation of the received signal.

WO 96/17565 discloses an apparatus for delivery of gas such as oxygen solution into a coronary artery comprising a high-pressure tubular housing defining a lumen.

It is the object of the invention to provide an apparatus for the preparation of a gas-enriched fluid to be delivered to a patient in which the priming volume is minimized.

This object is fulfilled by an apparatus having the features disclosed in claim 1. Preferred embodiments are defined in the dependent claims.

### SUMMARY OF THE INVENTION

The present invention may address one or more of the problems set forth above. Certain embodiments of the present invention are set forth below as examples. It should be understood that these aspects are presented merely to provide the reader with a brief summary of certain forms the invention might take and that these aspects are not intended to limit the scope of the invention. Indeed, the invention may encompass a variety of aspects that may not be set forth below, but which fall under the claims.

In one embodiment of the present invention, a system for the preparation and delivery of a gas-enriched fluid is provided. In applications involving the prevention of ischemia or the treatment of ischemic tissues, the system may be used for the preparation and delivery of an oxygen-enriched fluid including blood to a specific location within a patient's body. The system may include a circuit for oxygenating or enriching blood, e.g., increasing the level of dissolved oxygen in the blood. The system includes an apparatus that combines a gas-supersaturated fluid with blood to form a gas-enriched fluid, advantageously for regional or localized delivery. The gas-supersaturated fluid may include an oxygen-supersaturated physiologic liquid, and the blood to be enriched is blood withdrawn from the patient.

The system provided further includes assemblies for supplying controlled flows or supplies of the gas-supersaturated fluid and the blood. The system may include an elongated, generally tubular assembly including a central lumen and at least one end placeable within a patient body proximate a tissue site to be treated, the end including an outlet port for the gas-enriched fluid. The system may include a catheter defining a fluid pathway, including a proximal portion adapted for coupling to supplies of gas-supersaturated fluid and blood, and a distal portion defining a fluid pathway removably insertable within a patient's body, for infusing the gas-enriched fluid to predetermined sites.

The proximal portion of the catheter is adapted for coupiing to a supply of gas-supersaturated fluid, and includes a pump loop through which blood drawn from a blood inlet flows. The blood inlet comprises a porous side segment or axial sleeve defining the entry into an annular conduit that transitions into a lumen in fluid communication with the pump loop. The inlet is disposed along the portion of the catheter removably insertable within the patient's body. Upon insertion of the catheter through an access or opening, e.g., an introducer sheath, and upon its placement within the patient body, e.g., tip placement in or proximate the coronary ostium, the blood inlet is distal to the access sheath so as to permit blood from the patient to pass through and along the fluid path defined by the blood inlet, annular conduit, lumen and pump loop before combining with the gas-supersaturated fluid to form the gas-enriched fluid delivered to the patient via the catheter central lumen and outlet port.

The invention may be used in a method for the preparation and delivery of a gas-enriched fluid. In applications involving the prevention of ischemia or the treatment of ischemic tissues, the method may include the step of combining a gas-supersaturated fluid with blood to form a gas-enriched fluid. Advantageously, the gas-supersaturated fluid comprises an oxygen-supersaturated physiologic liquid in which oxygen is dissolved at concentrations normalized to standard temperature and pressure (STP) that equal or exceed the volume of the solvent. Examples of solvents which may be used include saline, lactated Ringer's, and other water-based physiologic solutions.

Further, the invention may be used in a method for delivering an oxygen-enriched fluid to a specific site within a patient's body. The method comprises raising the pO₂ level of the fluid to be supplied to the patient Where the fluid to be infused includes blood, the method may include the step of controlling or providing controlled amounts of the blood and oxygen-supersaturated fluid that are combined so as to produce an oxygen-enriched fluid for delivery to a specific predetermined site. Blood pO₂ levels may be maintained, adjusted, or otherwise controlled by controlling the flow rates or by providing controlled amounts of the blood and/or oxygen-supersaturated fluid. Thus, a blood-gas control method is provided.

Furthermore, delivery of the gas-enriched fluid advantageously occurs without the formation of clinically significant bubbles. To help minimize or eliminate the formation of clinically significant bubbles, the blood contact surfaces are exposed to or coated with blood proteins for some brief time interval, usually at least several minutes, before the start of infusion of oxygen-supersaturated fluid. Also, fluid contact surfaces are exposed to or pre-wetted with liquids, e.g., saline, ethanol and benzalkonium heparin, before use. The fluid contact surfaces also do not include any substance which promotes such bubble formation, e.g., hydrophobic surfaces that are difficult to wet, teflon, teflon-composite liners, silicone oils, etc. Hydrophillic fluid contact surfaces are typically useful.

The embodiments may be used in conjunction with angiographic or guiding catheters, arterial sheaths, and/or other devices used in angioplasty and in other interventional - cardiovascular procedures. The system may be used in applications involving one or more vascular openings, i.e., in either contralateral or ipsilateral procedures.

In contralateral procedures blood is withdrawn from the patient at a first location, e.g., the left femoral artery. The blood is enriched and then is returned to the patient at a second location proximate the tissue to be treated. Blood enrichment occurs as the blood pumped through the extracorporeal circuit or loop is combined with the gas-supersaturated fluid to form the gas-enriched fluid to be delivered. The catheter includes proximal and distal ends and a central lumen. The proximal end is adapted for the catheter to receive a supply of the gas-supersaturated fluid and to receive the blood. The distal end is removably insertable within a patient's body through a second location such as the patient's right femoral artery. The distal end includes at least one port in fluid communication with the central lumen and through which the gas-enriched fluid may exit. Further, the distal portion of the catheter may be adapted with a tip portion shaped so as to promote insertion of the device, such as through the same sheath used for interventional procedures like angioplasty, to specific predetermined locations within a patient's body. Examples of tip portion shapes which may be used include any of the standard clinically accepted tip configurations used with devices like guide catheters for providing access to and for holding in locations like the coronary ostium. Accordingly, the method may further include the step of positioning the portion of the distal end of the catheter including the fluid exit port at a predetermined location within a patient body proximate to the tissue to be treated.

In ipsilateral procedures, the system may be used along with one or more of any of a number of suitable, standard-size, clinically accepted guide catheters and/or introducer sheaths. The system, for example, may comprise a catheter, a catheter and guide catheter, or a catheter and sheath, for use within a guide catheter or introducer sheath used for the primary interventional procedure. In accordance with this embodiment of the present invention, blood is drawn between the catheter and guide catheter or sheath assemblies of the present invention, between the catheter assembly of the present invention and the guide catheter or introducer sheath used for the primary interventional procedure, or from the annular space between the guide catheter and the introducer sheath.

As described herein, the preferred gas-supersaturated fluid for use in accordance with the present invention is an oxygen-supersaturated fluid. However, other fluids may be used depending upon the circumstances involved in a particular desired application, such as, for example, supersaturated fluids in which one or more gases such as helium, nitrous oxide, carbon dioxide and air are dissolved. The oxygen-supersaturated fluid may include a dissolved oxygen volume normalized to standard temperature and pressure of between about 0.5 and about 3 times the volume of the solvent. The fluid is supplied to the system at a pressure of between about 1.7 MPa and about 34,5 MPa (250 p.s.i. and about 5000 p.s.i.). The exact pressure may vary depending upon the circumstances involved in a particular application. Further, the oxygen-supersaturated fluid supplied may be a sterile fluid which does not include gas, surface, or bubble nucleation sites at which clinically significant bubbles may form.

The catheter system of the present invention is typically sized in accordance with the circumstances involved in a particular application. In general, the sizes of the various system components will be on the order of the sizes of clinically accepted interventional cardiovascular devices. Usually, the extracorporeal loop of the present invention is less than four meters in total length. Thus, for example, where the system supports blood flow rates between 100 ml/min and 175 ml/min, the priming volume would be approximately 35 ml.

### BRIEF DESCRIPTION OF THE DRAWINGS

Further objects and advantages of the present invention will become apparent upon reading the following detailed description and upon referring to the accompanying drawings in which:
FIG. 1 is a schematic diagram of an exemplary embodiment of a catheter system in accordance with the present invention used in a contralateral interventional procedure.
FIG. 2 is a cross-sectional view of part of an exemplary embodiment of a catheter system including an angled blood flow path in accordance with the present invention.
FIG. 3 is a cross-sectional view of part of an alternate exemplary embodiment of a catheter system including an angled blood flow path in accordance with the present invention.
FIG. 4 is a cross-sectional view of part of another exemplary embodiment of a catheter system including a straight blood flow path in accordance with the present invention.
FIG. 4A is a cross-sectional view along line A-A in FIG. 4 of the exemplary embodiment of a catheter system including a straight blood flow path shown in accordance with the present invention.
FIG. 5 is a perspective view of part of an exemplary embodiment of a catheter system including an exemplary oxygen-supersaturated fluid outlet in accordance with the present invention.
FIG. 5A is a cross-sectional view along line A-A in FIG. 5 of the part of the exemplary embodiment of a catheter system including an exemplary oxygen-supersaturated fluid outlet shown in accordance with the present invention.
FIG. 5B is a view of the exemplary oxygen-supersaturated fluid outlet shown in FIG. 5 in accordance with the present invention.
FIG. 6 is a cross-sectional view of part of an exemplary embodiment of a catheter system including an integrated blood inlet introducer sheath.
FIG. 7 is a schematic diagram of an exemplary embodiment of a catheter system for use in an ipsilateral interventional procedure.
FIG. 8 is a cross-sectional view of part of an exemplary guide catheter including a liner used with an exemplary embodiment of a catheter system in accordance with the present invention.
FIG. 8A is a cross-sectional view of the exemplary guide catheter shown in FIG. 8, without the liner, in accordance with the present invention.
FIG. 9 is a cross-sectional view of part of an exemplary catheter system including an integral blood draw comprising a porous side blood inlet in accordance with the present invention.
FIG. 10 is a cross-sectional view of part of an exemplary catheter system including an integral blood draw comprising an axial blood inlet in accordance with the present invention.
FIG. 10A is a cross-sectional view along line A-A in FIG. 10 of the part of an exemplary catheter system shown in accordance with the present invention.
FIG. 11 is a view of part of an alternate exemplary catheter system including an integral blood draw comprising a porous side blood inlet in accordance with the present invention.
FIG. 11A is a cross-sectional view along line A-A in FIG. 11 of part of an alternate exemplary catheter system including an integral blood draw comprising a porous side blood inlet in accordance with the present invention.
FIG. 11B is a cross-sectional view along line B-B in FIG. 11 of part of an alternate exemplary catheter system including an integral blood draw comprising a porous side blood inlet in accordance with the present invention.
FIG. 11C is a cross-sectional view along line C-C in FIG. 11 of part of an alternate exemplary catheter system including an integral blood draw comprising a porous side blood inlet in accordance with the present invention.
FIG. 11D is a cross-sectional view along line D-D in FIG. 11 of part of an alternate exemplary catheter system including an integral blood draw comprising a porous side blood inlet in accordance with the present invention.
FIG. 12 is a cross-sectional view of an exemplary tip configuration of a catheter in accordance with the present invention.
FIG. 12A is a cross-sectional view along line A-A in FIG. 12 of an exemplary tip configuration of a catheter in accordance with the present invention.
FIG. 12B is a cross-sectional view along line B-B in FIG. 12 of an exemplary tip configuration of a catheter in accordance with the present invention.
FIG. 13 is a cross-sectional view of an alternate exemplary tip configuration of a catheter in accordance with the present invention.
FIG. 13A is a cross-sectional view along line A-A in FIG. 13 of an alternate exemplary tip configuration of a catheter in accordance with the present invention.
FIG. 13B is a cross-sectional view along line B-B in FIG. 13 of an alternate exemplary tip configuration of a catheter in accordance with the present invention.
FIG. 14 is a cross-sectional view of an alternate exemplary tip configuration of a catheter in accordance with the present invention.
FIG. 14A is a cross-sectional view of a further alternate exemplary tip configuration of a catheter in accordance with the present invention.
FIG. 15 is an isometric view of an exemplary bubble detector transducer in accordance with the present invention.
FIG. 15A is a partially exploded view of a portion of the bubble detector transducer shown in FIG. 15.

The present invention may be susceptible to various modifications and alternative forms. Specific embodiments of the present invention are shown by way of example in the drawings and are described herein in detail. It should be understood, however, that the description set forth herein of specific embodiments is not intended to limit the present invention to the particular forms disclosed. Rather, all modifications, alternatives, and equivalents falling within the scope of the invention as defined by the appended claims are intended to be covered.

### DETAILED DESCRIPTION OF SPECIFIC EMBODIMENTS

The description below illustrates embodiments of the present invention. For the sake of clarity, not all features of an actual implementation of the present invention are described in this specification. It should be appreciated that in connection with developing any actual embodiment of the present invention many application-specific decisions must be made to achieve specific goals, which may vary from one application to another. Further, it should be appreciated that any such development effort might be complex and time-consuming, but would still be routine for those of ordinary skill in the art having the benefit of this disclosure.

For the sake of clarity and convenience, the various embodiments are described herein in the context of interventional cardiovascular applications generally involving acute or transient ischemia or post-ischemic tissues. However, the present invention may also useful in other medical applications, such as cancer therapy (e.g., the delivery of oxygen-enriched fluids directly into poorly vascularized tumors during radiation or chemotherapy treatments), neurovascular applications (e.g., the treatment of stroke and cerebral trauma patients), lung support in trauma and lung disease patients, and wound care management.

Also, although the present invention may be used to raise oxygen levels, for example, in venous and arterial blood, in blood substitutes, e.g., perfluorocarbons, and in combinations thereof, for the sake of clarity and convenience reference is made herein only to arterial blood.

Further, the present invention also may be used in connection with drug fluid infusion therapies. Examples of drug fluids used in cardiovascular and neurological procedures which may be infused in accordance with the present invention include, without limitation, vasodilators (e.g., nitroglycerin and nitroprusside), platelet-actives (e.g., ReoPro and Orbofiban), thrombolytics (e.g., t-PA, streptokinase, and urokinase), antiarrhythmics (e.g., lidocaine, procainamide), beta blockers (e.g., esmolol, inderal), calcium channel blockers (e.g., diltiazem, verapamil), magnesium, inotropic agents (e.g., epinephrine, dopamine), perofluorocarbons (e.g., fluosol), crystalloids (e.g., normal saline, lactated ringers), colloids (albumin, hespan), blood products (packed red blood cells, platelets, whole blood), Na+/H+ exchange inhibitors, free radical scavengers, diuretics (e.g., mannitol), antiseizure drugs (e.g., phenobarbital, valium), and neuroprotectants (e.g., lubeluzole).

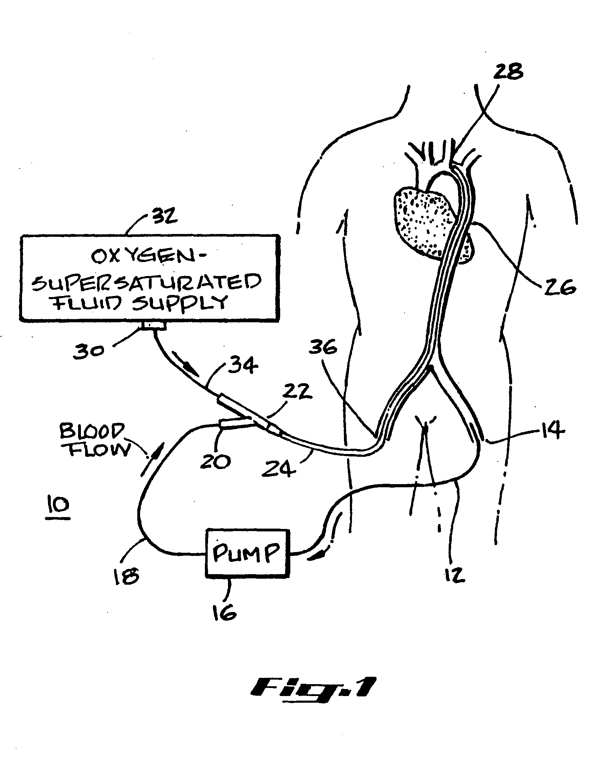

Turning now to the drawings, a system is provided in which blood is combined with an oxygen-supersaturated fluid to form an oxygen-enriched fluid that may be delivered to a particular predetermined area within a patient's body for the treatment of conditions such as tissue ischemia and post-ischemic tissues. As shown in Fig. 1, one embodiment of such a system **10** includes a blood draw **12** comprising a continuous fluid flow path between a vascular access site **14** of a patient body and a pump **16.** The selection of the vascular access site typically is made by a physician or caregiver and depends upon the circumstances surrounding the particular application involved. The particular vascular access site illustrated in Fig. 1 is the left femoral artery. The blood pump **16** may be one of the many commercially available and clinically accepted blood pumps suitable for use with human patients. An example of one such pump is the Model 6501 RFL3.5 Pemco peristaltic pump available from Pemco Medical, Cleveland, Ohio.

The pump **16** draws blood from the patient and provides a supply of blood via line 18 to the inlet **20** of catheter **22.** The flow characteristics of the blood will depend upon the - circumstances surrounding the particular application involved. Typically, the supply of blood to the blood inlet **20** of catheter **22** will be a controlled flow defined by the flow parameters selected by the caregiver. Factors influencing the determination of blood flow characteristics may include one or more of the many clinical parameters or variables of the blood to be supplied to the catheter or of the oxygen-enriched fluid to be delivered to the patient, e.g., the size of the patient, the percentage of overall circulation to be provided, hemolysis, hemodilution, pO₂, pulsatility, mass flow rate, volume flow rate, temperature, hemoglobin concentration and pH.

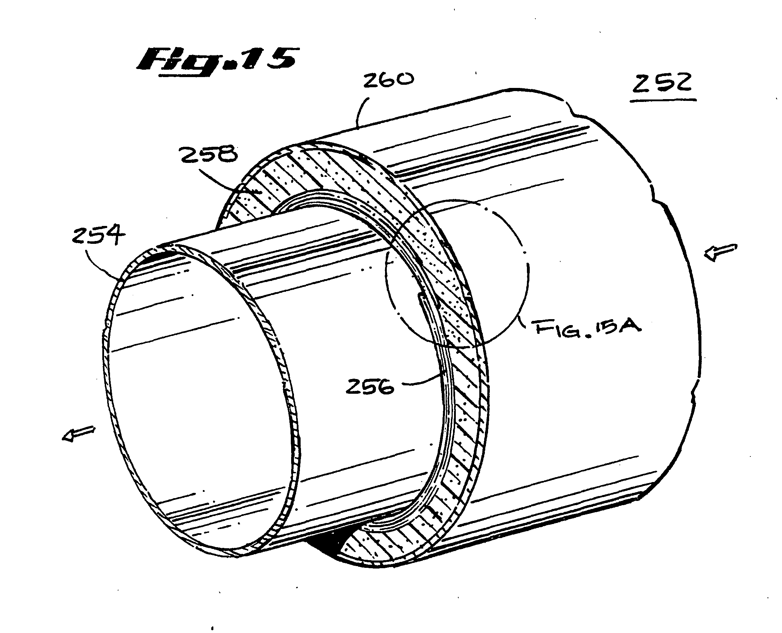

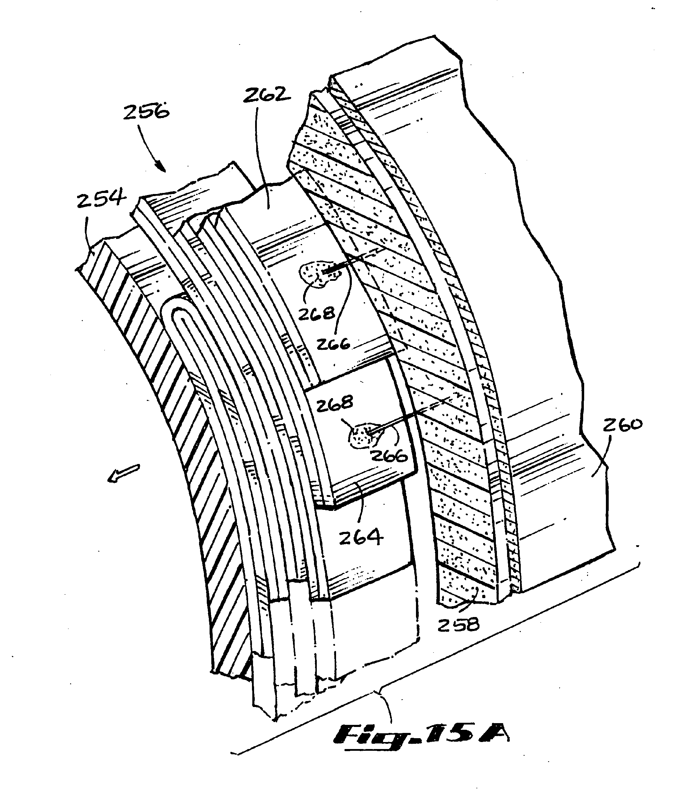

The system 10 may include one or more gas bubble detectors, at least one of which is capable of detecting the presence of microbubbles, i.e., bubbles with diameters of about 7 to 10 microns to about 200 microns (see, e.g., Fig. 12). In addition, the system may include one or more macrobubble detectors to detect larger bubbles, such as bubbles with diameters of 1 millimeter or more. Such macrobubble detectors may comprise any suitable commercially available detector, such as an outside, tube-mounted bubble detector including two transducers measuring attenuation of a sound pulse traveling from one side of the tube to the other. One such suitable detector may be purchased from Transonic Inc. of New York. The microbubble and macrobubble detectors provide the physician or caregiver with a warning of potentially clinically significant bubble generation. The system **10** also may include various conventional items, such as sensors, flow meters (which may also serve a dual role as a macrobubble detector), or other clinical parameter monitoring devices; hydraulic components such as accumulators and valves for managing flow dynamics; access ports which permit withdrawal of fluids; filters or other safety devices to help ensure sterility; or other devices that generally may assist in controlling the flow of one or more of the fluids in the system **10.** Advantageously, any such devices are - positioned within the system and used so as to avoid causing the formation of clinically significant bubbles within the fluid flow paths.

The catheter **22** includes a proximal portion **24** and a distal portion **26,** the distal portion being removably insertable within a patient body through a vascular access or opening **36.** The proximal portion **24** includes blood inlet **20.** Blood inlet **20** and line **18** may be adapted for releasably coupling, e.g., with a clinically accepted fluid connection apparatus such as a Luer lock, to enable the catheter **22** to receive the blood supplied via line **18.** The catheter **22** includes a lumen defining a continuous blood flow path from the blood inlet **20** to a fluid exit port proximate the distal tip **28** of catheter **22.**

The proximal portion **24** of catheter **22** also includes a fluid inlet port **30** adapted to couple to a supply **32** of oxygen-supersaturated fluid. The port **30** is in fluid communication with the supply **32** and with line **34.** Line **34** comprises one or more capillaries or other elongated generally tubular members including central lumens, either alone or in an array, each defining a continuous fluid flow path between port **30** and the blood flow path of the catheter **22.**

The oxygen-supersaturated fluid is usually supplied to port **30** in accordance with parameters specified and selected by the caregiver for the desired clinical indication. The flow of oxygen-supersaturated fluid is generally steady and continuous, although variable or intermittent flows may be used. Flow rates may range from about 0.1 cc/min to about 40 cc/mm, although particularly advantageous flow rates may be between about 2 cc/min and 12 cc/min. Oxygen concentrations may range from about 0.5 cc O₂ per cc physiologic solution to about 3 cc O₂ per-cc physiologic solution, although particularly advantageous concentrations may be about 1 cc O₂ per cc physiologic solution. The oxygen-supersaturated fluid is provided at a temperature such that when the fluid combines with blood to form the oxygen-enriched fluid to be infused, the oxygen-enriched fluid is about 37°C, i.e., system operation does not significantly affect patient blood temperature.

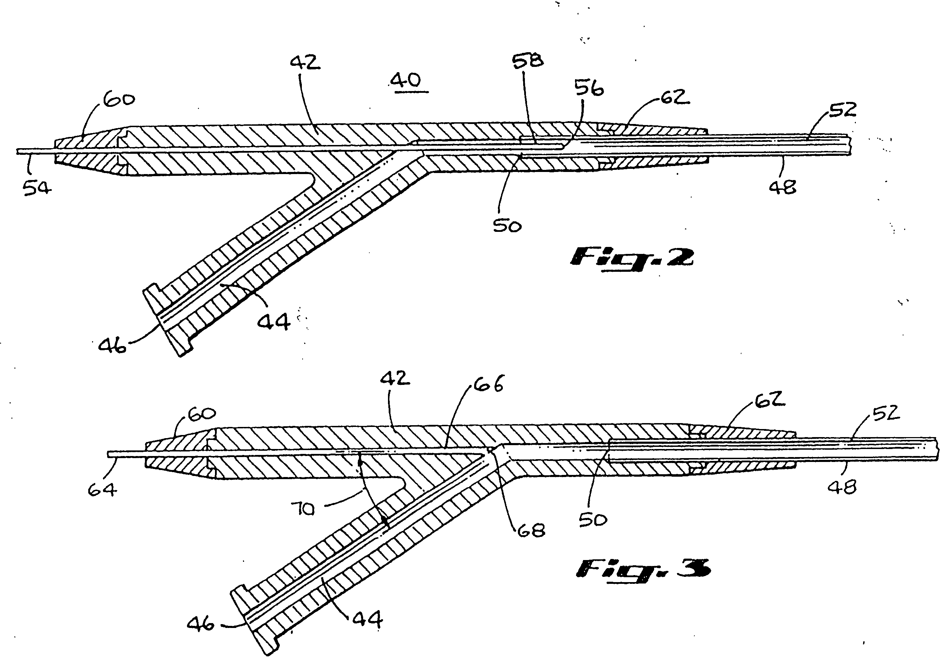

Figure 2 shows an embodiment in which catheter portion **40** comprises a section of the catheter in which the flows of blood and oxygen-supersaturated fluid combine. Catheter portion **40** comprises the proximal end of an elongated tubular member **48,** including a generally centrally disposed fluid return lumen **52,** disposed within a housing **42** including a blood inlet lumen **44.** Lumen **44** defines a continuous fluid flow path between catheter blood inlet port **46** and the proximal end **50** of fluid return lumen **52.** The distal portion of member **48** (not shown) is removably insertable within a patient body and includes the exit port through which fluid traveling within fluid return lumen **52** is delivered to a site within a patient's body.

Housing **42** typically comprises a biocompatable, molded polymeric material. The exact size and shape of housing **42** may vary depending upon the circumstances involved in a particular application. Figure 2, by way of example, shows a generally y-shaped configuration. The tubular member **48** may be integrally formed with housing **42.** However, tubular member **48** may comprise clinically approved polymeric tubing. The proximal end of member **48** is fixedly attached within housing **42.** Typically, the joining of housing **42** and member **48** is accomplished by solvent or adhesive bonding or insert or over molding.

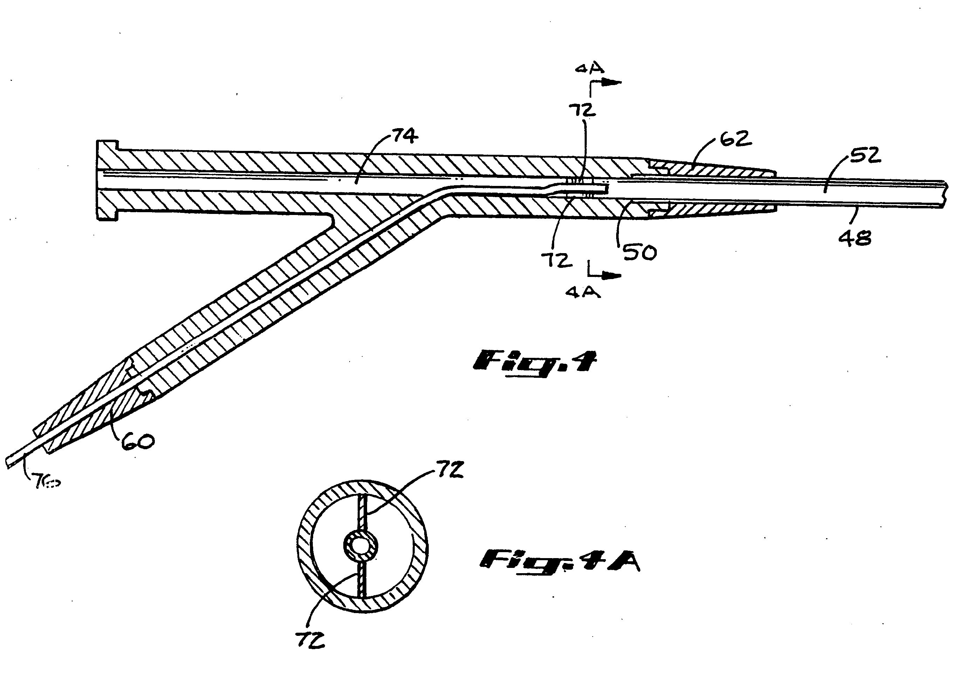

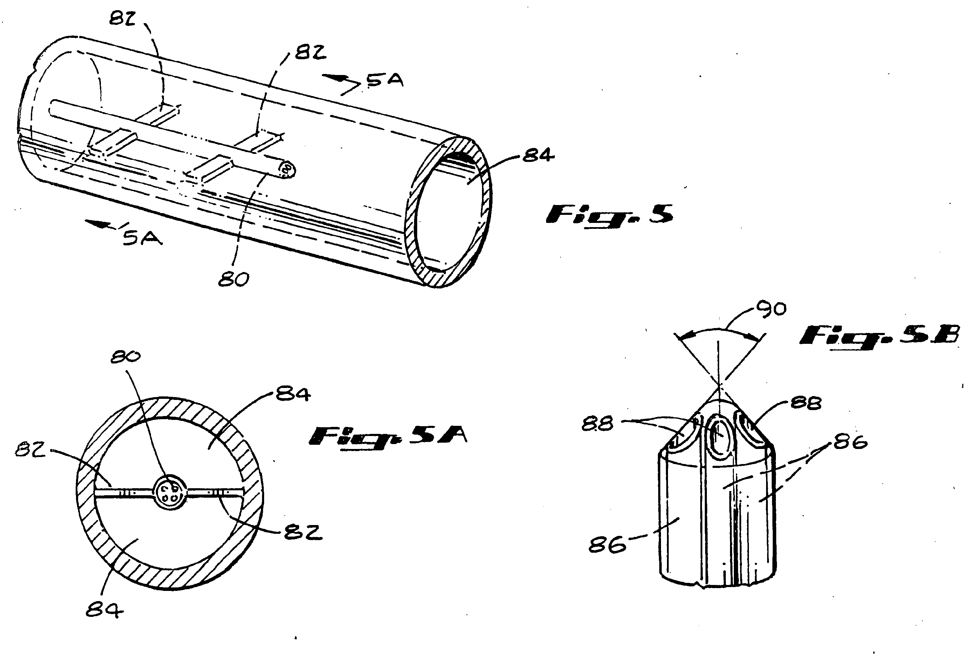

The size and shape of lumen **44** also may vary depending upon the circumstances involved in a particular application. Fig. 2, by way of example, shows a lumen **44** defining an angled blood flow path. However, a straight or curved blood flow path might also be used. See, e.g., Fig. 4. Advantageously, any difference between the inner diameter of the proximal end of member **48** and the diameter of lumen **44** at the proximal end **50** of fluid return lumen **52** is minimized or eliminated to promote smooth blood flow.

Catheter portion **40** also includes the distal portion of an oxygen-supersaturated fluid delivery line **54.** The line **54** comprises at least one elongated generally tubular member including a central lumen defining a fluid flow path between a fluid inlet port (not shown in Fig. 2) disposed at the proximal end of line **54** and fluid exit port **56** disposed at the distal end **58** of line **54.** Catheter portion **40** also may include one or more stress/strain relief assemblies **60, 62.**

The proximal end of line **54** may be adapted to releasably couple to a supply of oxygen-supersaturated fluid. The fluid exit port **56** may be disposed within the flow path defined by fluid return lumen **52.** Thus, a continuous fluid flow path is defined between the supply of oxygen-supersaturated fluid and a predetermined site within a patient's body proximate the distal end of fluid return lumen **52.**

The portions proximate to fluid exit port **56** of fluid return lumen **52** and of the distal end **58** of line **54** are generally straight, and their longitudinal axes approximately coincide, so that any difference in the direction of blood flow in lumen **52** proximate port **56** and the direction of exit fluid flow through port **56** is minimized or eliminated.

As shown in Fig. 2, fluid exit port **56** may be sufficiently downstream of the proximal end **50** of fluid return lumen **52** that the fluid exiting port **56** avoids any fluid flow disruption or non-laminar flow associated with the boundary between housing **42** and member **48** that might cause the formation of clinically significant gas bubbles. However, where any such flow disruptions or non-laminar flows are minimized or eliminated, the exit port for the oxygen-supersaturated fluid may be disposed upstream of the proximal end of the fluid return lumen. See, e.g., Figs. 3 and 4. Further, as shown in Fig. 3, in an alternate embodiment, an oxygen-supersaturated fluid line **64** may include a distal portion **66** including a fluid exit port **68** at the outer boundary of lumen **44.** For example, as shown in Fig. 3, the longitudinal axis of line **64** proximate port **68** and the longitudinal axis of the portion of lumen **44** downstream of port **68** advantageously coincide, while the longitudinal axis of line **64** proximate port **68** and the longitudinal axis of the portion of lumen **44** upstream of port **68** advantageously form an angle **70** comprising an acute angle which permits the smooth introduction of fluid from line **64** into the blood flow through lumen **44.** Advantageously, the angle **70** may be about thirty degrees, for instance.

As shown in Fig. 2, the portion of line **54** extending from housing **42** into the blood flow path may be rigid enough to comprise a cantilever-like member. However, as shown in Fig. 4, - the extending portion of the oxygen-supersaturated fluid flow line **76** also may comprise a more flexible member which tends to align itself naturally within blood flow lumen **74** along the path of least resistance. The distal end of line **76** may be supported in place or otherwise oriented within the lumen **74** by one or more wings **72** extending between the distal end of oxygen-supersaturated line **76** and the outer wall or boundary defining lumen **74.** The flexibility and positioning of the lines **54** and **76** depend upon the circumstances involved in a particular application, e.g., the material hardness, line profile, the number of capillaries making up the line, and the desired fluid exit location.

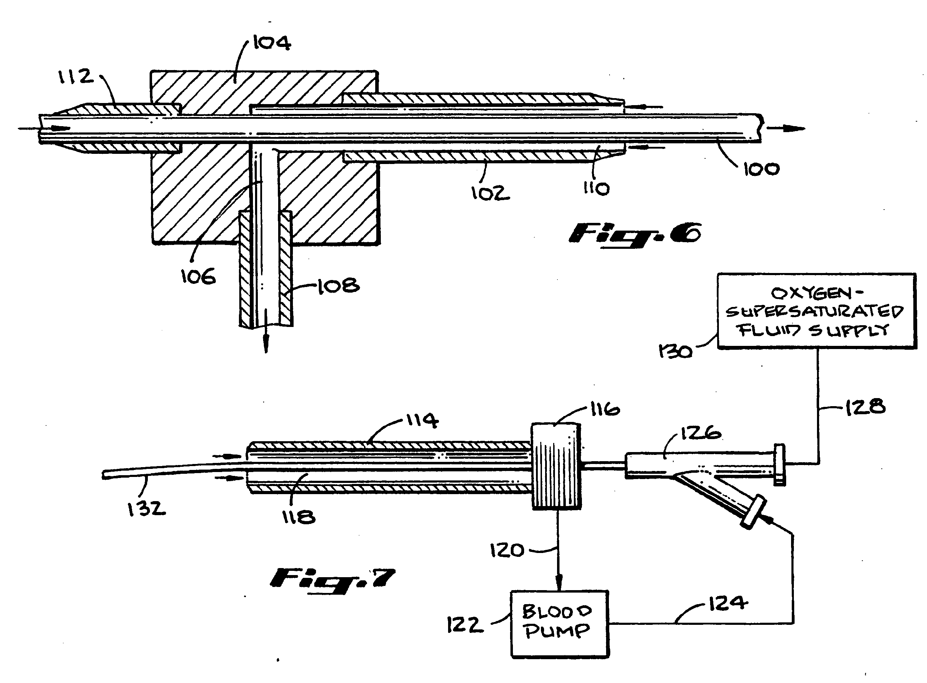

The oxygen-supersaturated fluid is injected so as to minimize or avoid altogether blood cell damage. An exemplary oxygen-supersaturated fluid outlet is shown in Fig. 5. The oxygen-supersaturated fluid line **80** is disposed within fluid delivery lumen **84.** The distal tip of line **80** is oriented within lumen **84** by one or more ribs or spacers **82** securing the distal portion of line **80.** The line **80** includes one or more capillaries **86** (see Fig. 5B) each including a central lumen **88** through which oxygen-supersaturated fluid flows. The configuration of the distal tip of line **80** advantgeously minimizes or eliminates flow disruptions resulting from the exit of fluid from each lumen **88** into the flow of blood within lumen **84.** By way of example, Fig. 5B shows a line **80** comprising four capillaries **86** and including a distal tip of generally conical shape. The ends of each capillary **86** may form an included angle **90** of about 52 degrees, for instance. The capillaries may be made of glass sheathed in polyimide, with ground and polished distal ends to help minimize or eliminate the formation of clinically significant bubbles and fluid flow disruptions. The line 80 may include four 100 micron inner diameter by 350 micron outer diameter tubes, for instance, potted together with epoxy at their proximal and distal ends. However, the inner diameter may be in the range of about 20 to about 1000 microns, with an inner diameter of about 100 to about 125 microns being particularly advantageous. Of course the exact size and shape of the distal end and tip of line 80 may vary depending upon the circumstances involved in a particular application. Examples of possible configurations include, without limitation, flat, blunt, squared, pencil-shaped, curved, parabolic, hyperbolic, and pyramidal.

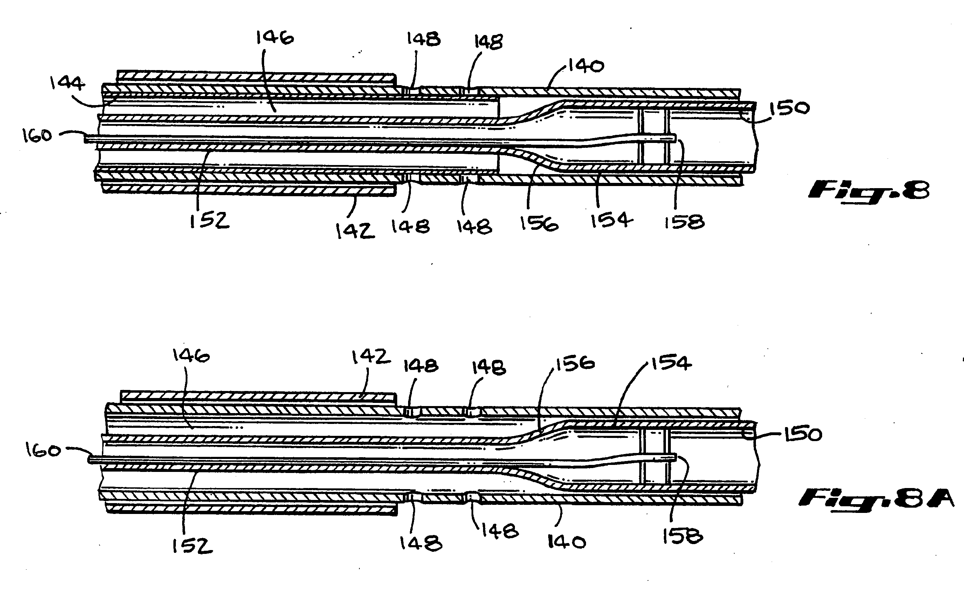

In the embodiment shown in Fig. 6, the catheter includes an oxygen-enriched fluid return line **100** and a blood draw assembly comprising a sheath **102** and housing **104.** The housing **104** includes a lumen **106** which forms a blood flow path between the lumens of sheath **102** and tube **108.** Tube **108** comprises the line which supplies blood to the blood pump (not shown in Fig. 6) of the system. The line **100** is generally centrally disposed through the housing **104** and within the central lumen of the sheath **102.** Upon insertion of the distal portion of line **100** into a patient's body, the sheath **102** is positioned within a vascular access sheath, guide catheter, or other such access device, so that blood from the patient may enter the annular space **110** between the outer wall of line **100** and the inner wall of sheath **102.** The proximal end of line **100** may include a stress/strain relief assembly **112.**

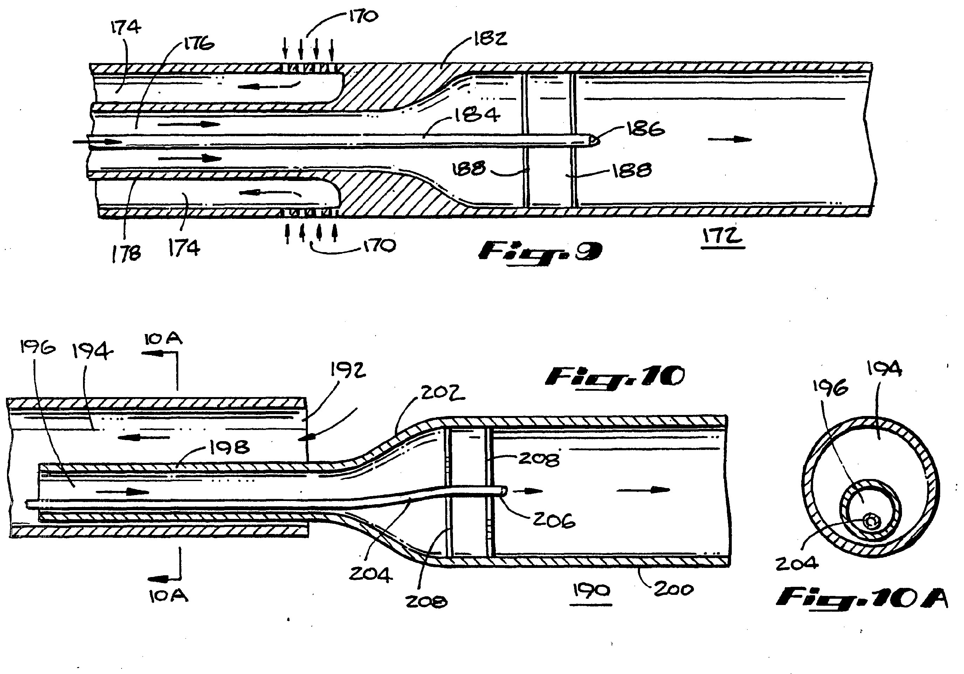

Figure 7 is a schematic diagram illustrating a use of the catheter system with a separate arterial access sheath **114.** The sheath **114** may be one of the many sizes and types of clinically accepted arterial access sheaths suitable for use in interventional cardiovascular procedures. The proximal end **116** of sheath **114** is adapted with a seal or other such device which permits the insertion of catheters, guidewires, or other interventional devices through the sheath **114** and into the body without unnecessary loss of blood. Blood drawn through the lumen **118** of sheath **114** travels via line **120** to blood pump **122** before being enriched and returned to the body. The blood returns via line **124** and the catheter portion **126,** where the blood flow combines with a flow of oxygen-supersaturated fluid delivered via line **128** from a supply **130** to form the oxygen-enriched fluid delivered to the patient via the distal portion **132** of the catheter system.

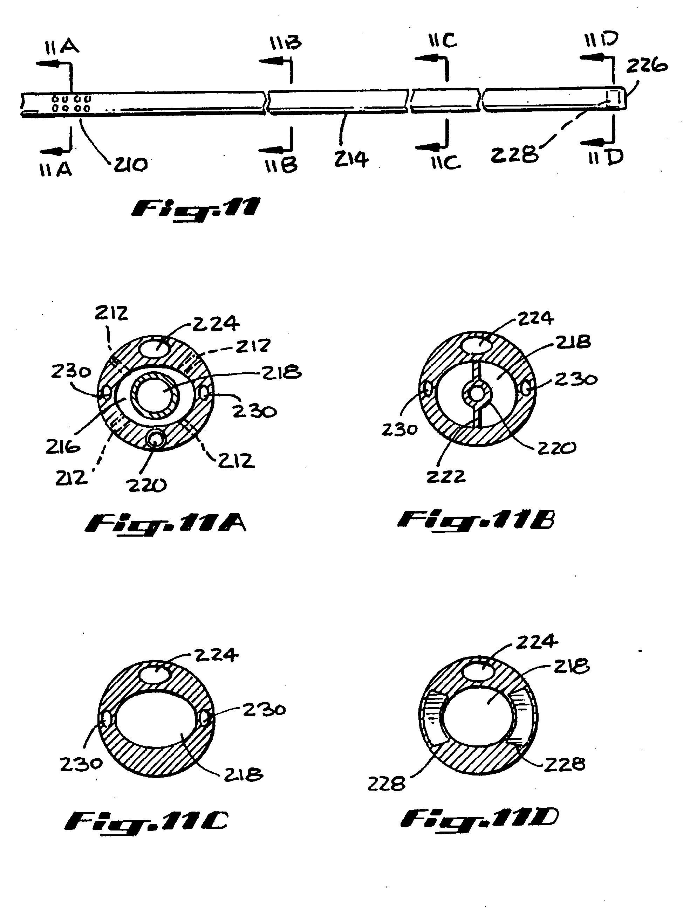

Figures 8 and 8A show an alternate embodiment of the catheter system. In accordance with this embodiment, a guide catheter **140** including a distal tip (not shown) removably insertable within a patient's body is placed through an outer arterial access sheath **142.** The sheath **142** may be any one of the many types of clinically accepted sheaths typically used in interventional cardiovascular procedures to gain access to a patient's vasculature. The guide catheter **140** includes a liner **144.** The liner **144** is deformable and may be either partially (not shown) or completely (see Fig. 8A) removed. When a positive pressure is applied to lumen **146** of guide catheter **140,** e.g., when angiographic dyes are introduced into the lumen **146,** and when the liner **144** is in place covering the blood inlet holes **148** through the wall of guide catheter **140,** the liner **144** presses against and closes inlet holes **148.** When a negative pressure is applied to lumen **146** of guide catheter **140,** e.g., during the withdrawal of blood from the patient, the liner deforms as necessary to allow the entry of blood into lumen **146** through inlet holes **148.**

To facilitate the draw of blood from the patient, the fluid return line **150** may include a proximal portion **152** having smaller internal and external diameters than the distal portion **154** of line **150.** When the distal tip of line **150** (not shown) is in place within the patient's body proximate a predetermined site, the transition region **156** is disposed downstream of blood inlet holes **148** so as to permit the entry of blood into lumen **146.** The transition region **156** comprises a section of the line **150** in which the external and internal diameters of the line **150** increase along its length. To minimize or eliminate the formation of clinically significant bubbles, downstream of transition region **156** the flow of blood in line **150** combines with oxygen-supersaturated fluid exiting at the end **158** of oxygen-supersaturated fluid supply tube **160.** As described herein (see, e.g., Fig. 5), the tube **160** may comprise either a single capillary or tube, or a bundle of capillaries or tubes.

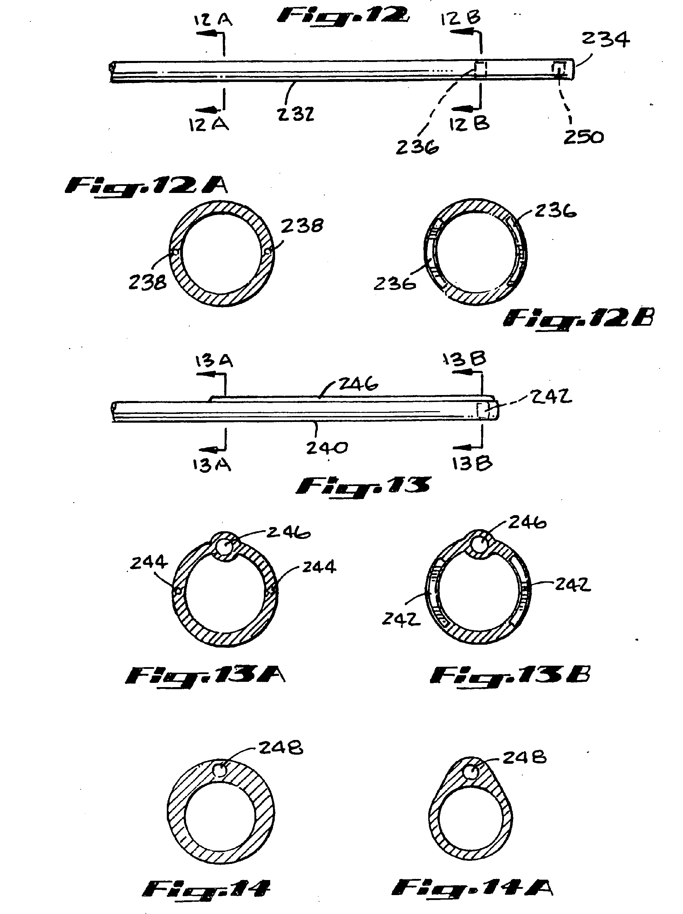

Figure 9 describes part of a catheter system including an integral blood draw comprising an annular porous side blood inlet **170.** The catheter **172** may be used along with a guide catheter or access sheath (not shown in Fig. 9). Blood is drawn from the patient and through the inlet **170** into catheter outer lumen **174.** From there, the blood circulates through a blood pump and is delivered back to the patient via catheter inner lumen **176.** As shown in Fig. 9, the inner lumen **176** includes a proximal portion **178** having relatively smaller internal and external diameters than distal portion, and a transition region **182** joining the two portions. Oxygen-supersaturated fluid supply tube **184** includes a fluid exit port **186** disposed within the distal portion of catheter **172.** The fluid exiting tube **184** enters the blood flow within lumen **176** downstream of any sharp pressure drops or other flow disturbances associated with the increase in the inner diameter of lumen **176** in the transition region **182.** Ribs or wings **188** or another such centering device may be used to hold the distal portion of tube **184** in place.

In an alternate embodiment, as shown in Fig. 10, the integral blood inlet of a catheter **190** comprises an axial blood inlet **192.** Blood from the patient is drawn from the interior of a guide catheter or sheath, or directly from the patient's vasculature, through the inlet **192,** and into catheter outer lumen **194.** From there the blood travels through a blood pump (not shown) and is returned to the patient via inner lumen **196.** As shown in Fig. 10, the inner lumen **196** includes a proximal portion **198** having relatively smaller internal and external diameters than distal portion **200,** and a transition region **202** joining the two portions. Oxygen-supersaturated fluid supply tube **204** includes a fluid exit port **206** disposed within the distal portion **200** of catheter **190,** and ribs or wings **208** may be used to hold the distal portion of tube **204** in place. The proximal portion **198** of inner lumen **196** may be fixed within the interior of outer lumen **194** by a further set or ribs or wings or other similar positioning device. As shown in Fig. 10, the proximal portion **198** of lumen **196** is free to naturally follow a path of least resistance through lumen **194.** The proximal portion of tube **204** likewise may be secured within lumen **196** or be free as shown.

Figures 11 and 11A-D show part of an alternate exemplary catheter system including an integral blood draw. A porous side blood inlet **210** comprising a plurality of channels **212** through the outer wall of catheter **214** allows blood from the patient to enter outer lumen **216.** The blood in lumen **216** passes through a blood pump (not shown in Fig. 11) before returning to the patient via lumen **218.** Within lumen **218** the blood combines with an oxygen-supersaturated fluid supplied via a tube **220.** As shown in Fig. 11, the tube **220,** along at least a portion of its length, may be disposed within the lumen **218.** The distal tip of the tube **220** is positioned along the longitudinal axis of lumen **218** and secured in place by one or more centering fins or spacers. **222.**

The catheter **214** also may include a lumen **224** through which blood proximate the distal tip **226** of the catheter **214** may be drawn, e.g., to provide a blood sample for use in determining blood pO₂ or in the monitoring of others clinical parameters, to ascertain blood pressure at the distal end of the catheter, etc.

The distal tip **226** of catheter **214** includes an ultrasonic bubble detector **228** or similar assembly for detecting the presence of clinically significant bubbles in the oxygen-enriched fluid delivered to the patient body via lumen **218.** Accordingly, the catheter **214** may also include one or more lumens **230** within which leads coupled to the bubble detector **228** are disposed.

Figures 12-14 illustrate alternate exemplary tip configurations of a catheter. Figure 12 shows a straight tip portion **232** including a bubble detector **236** disposed proximate the tip **234** of the catheter. Bubble detector leads **238** comprising one or more pairs of insulated wires or coaxial wires may be disposed as shown for example in Fig. 12A within the catheter side wall. Figure 13 illustrates a straight tip portion **240** including a dual transducer bubble detector **242,** bubble detector leads **244,** and a monorail guide lumen **246** through which a guidewire (not shown) may be thread to assist in the placement of the catheter within a patient body. One transducer of a dual transducer bubble detector typically emits an acoustic pulse which is received by the other transducer, and the presence or absence of bubbles is determined by measuring the attenuation of the received signal. Figures 14 an 14A illustrate alternate configurations of a catheter tip including a monorail guide lumen **248.** The catheter tip as shown in the drawings may include a relatively uniform wall thickness and be of generally circular cross section (Fig. 14), or it may have a varying wall thickness and assume a more tear-drop shape (Fig. 14A).

As shown in Fig. 12, a catheter tip also may include a radiopaque band **250** to aid the physician or caregiver in placing the device. Typically, the band **250** comprises one or more metals or metal alloys, e.g., platinum, gold, tungsten, and iridium, and platinum-indium and other high density materials that are visible under fluoroscopy.

A further exemplary bubble detector transducer **252** is shown in FIGS. 15 and 15A. The transducer **252,** which may be bonded or otherwise fixedly attached to or within the distal portion of the catheter, may include a single transducer operable in a pulse echo mode, i.e., it can send out an acoustic pulse and sense its reflection. The transducer **252** includes an inner sleeve **254,** a layer of wrapped piezo-electric film **256,** a metal band **258,** and an outer jacket **260.** The inner sleeve **254** comprises a polyimide sleeve. The wrapped film **256** comprises a metallized polyvinylidenefluoride (PVDF) film including a conductive upper layer **262,** a conductive lower layer **264,** and an insulative inner layer separating the upper and lower layers. As shown in the drawings, the metallized film is folded and then wrapped at least once (typically about two to five times) about the inner sleeve **254.** This folding permits the use of thinner film that exhibits higher capacitance and lower impedance than a more conventional transducer so that the wire leads **266,** which are long, thin wires, may be driven better. Wire leads **266** are connected to the outermost surfaces of the layers **262, 264** with a conductive epoxy **268.** The band **258** may comprise a foil of aluminum or a band of a metal which reflects the outward acoustic pulse. The band **258** comprises a radiopaque material so that the band may also act as a marker to promote placement of the device. The outer jacket **260** may be made of pebax or another suitable material which provides support to the structure. It should further be understood that the bubble detector illustrated in Figs. 15 and 15A is advantageously a compact device, e.g., air spaces between the layers of the bubble detector are minimized or eliminated. For example, any air voids may be filled with an epoxy or other suitable filler material.

The electronic circuitry **271** associated with the bubble detector transducer **252** causes the transducer **252** to emit ultrasonic pulses typically in the range of 20-30 MHz. In the pulse echo mode, these ultrasonic pulses are transmitted in a pulse train having a frequency of about 20-50 kHz. Thus, approximately ten reflections may be sampled from a single bubble as it passes by the transducer **252,** which is about 1 to 1.5 millimeters in length. After the pulses of ultrasonic energy are delivered during a positive portion of this duty cycle, the circuitry **271** waits approximately 0.5 microseconds to allow for a "ring down" period. Then, the reflected signals may be measured, typically for at least three bounces, before the next ultrasonic signals are transmitted.

The present invention has been described in terms of exemplary embodiments. In accordance with the present invention, the operating parameters for the system may be varied, typically with a physician or caregiver specifying and selecting them for the desired clinical indication. Further, it is contemplated that other embodiments, which may be readily devised by persons of ordinary skill in the art based on the teachings set forth herein, may be within the scope of the invention which is defined by the appended claims. The present invention may be modified and practiced in different but equivalent manners that will be apparent to those skilled in the art having the benefit of the teachings set forth herein.

No limitations are intended to the details or construction or design shown herein, other than as described in the claims appended hereto. Thus, it should be clear that the specific embodiments disclosed above may be altered and modified, and that all such variations and modifications are within the scope of the present invention as set forth in the claims appended hereto.

## Claims

1. An apparatus for the preparation and delivery of a gas-enriched fluid, in particular an oxygen-enriched fluid including blood, to a patient's body, the apparatus comprising:
means (32) for supplying a first fluid comprising a gas-supersaturated liquid and in particular an oxygen-supersaturated physiologic liquid;
means (16, 18) for supplying a second fluid; and
combining means (22) for combining the first and second fluids to form the gas-enriched fluid;
wherein said means (32) for supplying a first fluid being configured to supply said gas-supersaturated liquid at a pressure of between about 1.7 MPa (= 17 bar = 250 psi) and about 34,5 MPa (= 345 bar = 5000 psi) as a fluid not including clinically significant bubbles,
said means (16, 18) for supplying the second fluid includes a pump (16) for drawing blood from a patient as the second fluid, and
the combining means (22) is a catheter portion (40) of a catheter (22), the catheter portion (40) having a housing (42) in a Y-shaped configuration and including a central lumen in fluid communication with the means for supplying the first and second fluids.

2. The apparatus of claim 1, further comprising a microbubble detector (252) disposed proximate the distal end of the catheter.

3. The apparatus of any one of claims 1 or 2, wherein the catheter comprises:
the housing (42) having therein a first fluid passageway having a first end (60) and a second end (62) and a second fluid passageway having a first end (46) and a second end, the second end of the second fluid passageway intersecting the first fluid passageway at a junction between the first and second ends (60, 62) of the first fluid passageway, the first end (60) of the first fluid passageway being adapted to receive a first line (54) for supplying the gas-supersaturated fluid, and the first end (46) of the second fluid passageway being adapted to receive a supply of the second fluid, the first line (54) having an outlet (56) positioned in a substantially laminar flow region of the first fluid passageway, wherein the first line (54) comprises a plurality of wings (72) for supporting the first line (54) within the first fluid passageway.

4. The apparatus of claim 3, wherein the first fluid passageway is angled relative to the second fluid passageway at an acute angle (70).

5. The apparatus of any one of claims 1 to 4, comprising a second catheter (12) having a proximal end and a distal end, the proximal end being coupled to an inlet of the pump (16) , and the distal end being adapted to be positioned within a patient's body for delivering the second fluid from the patient's body to the pump (16), and comprising a tube (18) coupled between an outlet of the pump (16) and the first end of the second fluid passageway for delivering the second fluid to the second fluid passageway.

## Patentansprüche

1. Vorrichtung für die Herstellung und Abgabe einer mit Gas angereicherten Flüssigkeit, insbesondere einer mit Sauerstoff angereicherten Flüssigkeit, einschließlich Blut, in den Körper eines Patienten, wobei die Vorrichtung umfaßt:
eine Einrichtung (32) zum Zuführen einer ersten Flüssigkeit, die eine mit Gas übersättigte Flüssigkeit und insbesondere eine mit Sauerstoff übersättigte physiologische Flüssigkeit umfaßt;
eine Einrichtung (16, 18) zum Zuführen einer zweiten Flüssigkeit; und
eine Vereinigungseinrichtung (32) zum Vereinigen der ersten und zweiten Flüssigkeiten, um die gasangereicherte Flüssigkeit zu bilden;
wobei die Einrichtung (22) zum Zuführen der ersten Flüssigkeit derart aufgebaut ist, daß sie die mit Gas übersättigte Flüssigkeit mit einem Druck von etwa 1,7 MPa (= 17 bar = 250 psi) und etwa 34,5 MPa (= 345 bar = 5000 Pfund psi) als eine Flüssigkeit, die keine klinisch wesentlichen Blasen enthält, zuführt,
wobei die Einrichtung (16, 18) zum Zuführen der zweiten Flüssigkeit eine Pumpe (16) umfaßt, um Blut aus einem Patienten als die zweite Flüssigkeit zu saugen, und
die Vereinigungseinrichtung (22) ein Katheterabschnitt (40) eines Katheters (22) ist, wobei der Katheterabschnitt (40) ein Gehäuse (42) mit einem Y-förmigen Aufbau hat und ein zentrales Lumen in Flüssigkeitsverbindung mit der Einrichtung zum Zuführen der ersten und zweiten Flüssigkeiten umfaßt.

2. Vorrichtung nach Anspruch 1, die ferner eine Mikrobläschen-Erfassungsvorrichtung (252) umfaßt, die benachbart zu dem distalen Ende des Katheters angeordnet ist.

3. Vorrichtung nach einem der Ansprüche 1 oder 2, wobei der Katheter umfaßt:
das Gehäuse (42), das einen ersten Flüssigkeitsdurchgang mit einem ersten Ende (60) und einem zweiten Ende (62) darin und einen zweiten Flüssigkeitsdurchgang mit einem ersten Ende (46) und einem zweiten Ende hat, wobei das zweite Ende des zweiten Flüssigkeitsdurchgangs den ersten Flüssigkeitsdurchgang an einer Verbindung zwischen den ersten und zweiten Enden (60, 62) des ersten Flüssigkeitsdurchgangs schneidet, wobei das erste Ende (60) des ersten Flüssigkeitsdurchgangs geeignet ist, eine erste Leitung (54) zum Zuführen der mit Gas übersättigten Flüssigkeit aufzunehmen, und das erste Ende (46) des zweiten Flüssigkeitsdurchgangs geeignet ist, eine Zuführung der zweiten Flüssigkeit aufzunehmen, wobei die erste Leitung (54) einen Auslaß (56) hat, der in einem im wesentlichen laminaren Strömungsbereich des ersten Flüssigkeitsdurchgangs positioniert ist, wobei die erste Leitung (54) eine Vielzahl von Flügeln (72) zum Halten der ersten Leitung (54) innerhalb des ersten Flüssigkeitsdurchgangs umfaßt.

4. Vorrichtung nach Anspruch 3, wobei der erste Flüssigkeitsdurchgang relativ zu dem zweiten Flüssigkeitsdurchgang in einem spitzen Winkel (70) gewinkelt ist.

5. Vorrichtung nach einem der Ansprüche 1 bis 4, die einen zweiten Katheter (12) mit einem proximalen Ende und einem distalen Ende umfaßt, wobei das proximale Ende mit einem Einlaß der Pumpe (16) verbunden ist und das distale Ende geeignet ist, innerhalb des Körpers eines Patienten positioniert zu werden, um die zweite Flüssigkeit aus dem Körper des Patienten an die Pumpe (16) abzugeben, und ein zwischen einen Auslaß der Pumpe (16) und das erste Ende des zweiten Flüssigkeitsdurchgangs gekoppeltes Rohr umfaßt, um die zweite Flüssigkeit an den zweiten Flüssigkeitsdurchgang abzugeben.

## Revendications

1. Appareil pour la préparation et l'administration d'un fluide enrichi en gaz, en particulier un fluide enrichi en oxygène incluant du sang, au corps d'un patient, l'appareil comprenant :
des moyens (32) pour fournir un premier fluide comprenant un liquide sursaturé en gaz et en particulier un liquide physiologique sursaturé en oxygène ;
des moyens (16, 18) pour fournir un deuxième fluide ; et
dés moyens de combinaison (22) pour combiner les premier et deuxième fluides afin de former le fluide enrichi en gaz;
dans lequel les dits moyens (32) de fourniture d'un premier fluide sont configurés pour fournir le dit liquide sursaturé en gaz sous une pression comprise entre 1,7 MPa (= 17 bars = 250 psi) environ et 34,5 MPa (= 345 bars = 5000 psi) environ, sous la forme d'un fluide ne contenant pas de bulles cliniquement significatives,
les dits moyens (16, 18) pour fournir le deuxième fluide comprennent une pompe (16) pour prélever le sang d'un patient comme deuxième fluide, et
les moyens de combinaison (22) sont une partie de cathéter (40) d'un cathéter (22), la partie de cathéter (40) ayant un corps (42) en forme de Y et comportant une lumière centrale en communication de fluide avec les moyens de fourniture des premier et deuxième fluides.

2. Appareil selon la revendication 1, comprenant en outre un détecteur de microbulles (252) disposé près de l'extrémité distale du cathéter.

3. Appareil selon une quelconque des revendications 1 ou 2, dans lequel le cathéter comprend :
le corps (42) ayant intérieurement un premier passage de fluide qui a une première extrémité (60) et une deuxième extrémité (62), et un deuxième passage de fluide qui a une première extrémité (46) et une deuxième extrémité, la deuxième extrémité du deuxième passage de fluide étant en intersection avec le premier passage de fluide à l'endroit d'une jonction entre les première et deuxième extrémités (60, 62) du premier passage de fluide, la première extrémité (60) du premier passage de fluide étant prévue pour recevoir une première ligne (54) de fourniture du fluide sursaturé en gaz, et la première extrémité (46) du deuxième passage de fluide étant prévue pour recevoir une alimentation du deuxième fluide, la première ligne (54) ayant une sortie (56) située dans une région d'écoulement sensiblement laminaire du premier passage de fluide,
dans lequel la première ligne (54) comprend une pluralité d'ailettes (72) pour supporter la première ligne (54) dans le premier passage de fluide.

4. Appareil selon la revendication 3, dans lequel le premier passage de fluide est incliné par rapport au deuxième passage de fluide suivant un angle aigu (70).

5. Appareil selon une quelconque des revendications 1 à 4, comprenant un deuxième cathéter (12) ayant une extrémité proximale et une extrémité distale, l'extrémité proximale étant connectée à une entrée de la pompe (16), et l'extrémité distale étant prévue pour être positionnée dans le corps d'un patient pour amenée du deuxième fluide du corps du patient à la pompe (16), et comprenant un tube (18) connecté entre une sortie de la pompe (16) et la première extrémité du deuxième passage de fluide pour amenée du deuxième fluide au deuxième passage de fluide.
